# EUROPEAN PATENT APPLICATION

(11) **EP 4 343 786 A1**
(43) Date of publication of application: **27.03.2024**
(21) Application number: 22196449.7
(22) Date of filing: 20.09.2022
(51) Int. Cl.: G16H 50/20, G16H 30/40

(54) **BUILDING A MACHINE-LEARNING MODEL TO PREDICT SEMATIC CONTEXT INFORMATION FOR CONTRAST-ENHANCED MEDICAL IMAGING MEASUREMENTS**

(71) Applicant: Siemens Healthineers AG, 91301 Forchheim (DE)
(72) Inventor: Datar, Manasi, 99084 Erfurt (DE); Kraus, Martin, 90765 Fürth (DE); Neumann, Dominik, 91052 Erlangen (DE)
(74) Representative: Siemens Healthineers Patent Attorneys

(57) **Abstract**

A machine-learning model is pre-trained in an unsupervised manner to predict time-related information based on data obtained from a contrast-enhanced medical imaging measurement. This pre-trained machine-learning model is then used to build another machine-learning model to predict semantic context information for images determined from the contrast-enhanced medical imaging measurement.

## Description

Various examples of the disclosure generally relate to contrast-enhanced (CE) medical imaging. Various examples of the disclosure specifically relate to training of a machine-learning (ML) model in CE multi-phase imaging.

Contrast-enhanced (CE) imaging uses the diffusion and perfusion characteristics of contrast agents for improved visualization of normal or abnormal regions of interest (ROI) compared to the surrounding anatomy. This process normally involves the acquisition of a series of images of different distribution phases of the ROI before and following the administration of a contrast agent in a chronological order. Some examples of CE imaging techniques include lesion/tumor characterization for cancer staging, therapy or treatment planning using CE Computed Tomography (CT), diagnosis of blood vessel conditions (aneurysms, blockages) using CT angiography, or detection and characterization of brain abnormalities using CE Magnetic Resonance Imaging (MRI).

Detection and segmentation of ROIs from CE images is usually a tedious process involving an exhaustive, time consuming examination of individual images associated with different slices through an examination region and for each distribution phase by an expert. This can lead to uncertainty and errors. The visibility of the structures inside the ROI varies across different distribution phases based on the underlying diffusion of the contrast agent. Variability in clinical protocols used to acquire different distribution phases (hereinafter, also simply phases) leads to a lack of standardization and further contributes to subjectivity in interpretation.

Conventional solutions for detecting ROIs in images are based on supervised learning methods such as Machine Learning (ML) techniques including phase-agnostic detection, where the ML model is trained on all available phases. The high variability in the appearance of the ROI in individual phases, and between different phases, makes it difficult for such methods to converge. Methods combining detections from models trained on individual phases are known but suffer from the lack of availability of large cohorts of individual phases. Further, three-dimensional (3D) models may contribute to improving detection/segmentation by exploiting volumetric, highly detailed spatial and structural information. However, the large number of parameters in 3D models implies that they are slow to converge during training and have a slow inference speed.

All these conventional approaches rely on a large number of expert annotations of training images as a reference standard to train DL models, which may be time consuming and expensive to acquire.

Therefore, the idea of the presented approach is to provide advanced techniques for training a ML model for determining semantic context information for a contrast-enhanced medical image, which overcome or mitigate at least some of the limitations and disadvantages mentioned.

This task is solved by the features of the independent claims. Further advantageous examples are included in the dependent claims.

In the following, the solution according to the invention is described with regard to the claimed methods as well as with regard to the claimed computing devices, computer programs, storage media, and medical systems, wherein features, advantages, or alternative embodiments can be assigned to the other claimed objects and vice versa. For example, the claims related to the computing devices, computer programs and storage media can be improved with features described in the context of the methods. Further, the methods for applying the ML model may be improved with at least some features described in the context of training the ML model.

A computer-implemented method for building an ML model that predicts semantic context information from at least one inference image that is acquired using a CE medical imaging system is disclosed.

This ML model that predicts the semantic context information is built using at least a part of another ML model. This other ML model based on which the ML model is built is pre-trained to predict time-related information.

It has been found that such pre-training to predict time-related information (rather than the semantic context information) can simplify the training process of the pre-trained ML model. Thus the pre-trained ML model serves as a suitable starting point for building the ML model that eventually predicts the semantic context information.

Specifically, an unsupervised pre-training of the pre-trained ML model becomes possible.

The unsupervised pre-training can mean that manual annotations of certain spatial regions in the pre-training images are not required. A domain expert may not be required to generate ground truth. Self-learning is possible.

To distinguish between those two ML models, the ML model that predicts the time-related information is referred to as "ML model" (or, in its trained state, as "pre-trained ML model"); while the ML model that predicts the semantic context information is referred to as "further ML model".

Since the further ML model is built based on at least a part of the ML model, the presented approach may be understood as a two-step approach where first (in an upstream processing step) the ML model is (pre-)trained and then this ML model is used (in a downstream processing step) to build the further ML model. Thus, the further ML model can be built upon the ML model.

It has been found that a higher accuracy in the prediction of the further ML model can be achieved by such scenarios. In particular, by using unsupervised training for the ML model and building the further ML model based on the accordingly pre-trained ML model, a larger information set for the training can be considered without expensive manual labeling. Further, by predicting time-related information, the ML model is trained under consideration of the pharmacokinetics of the contrast agent. Thus, the ML model can extract relevant features that are associated with the phase-dependent appearance of certain region of interests (ROIs) or anatomy features in the CE images. Semantic context information can thus be more reliably determined based on such embedded features that take into consideration the phase-dependent change of the appearance of the CE images.

In various examples, the disclosed techniques may be used in CE imaging techniques which employ distribution, for example diffusion and/or perfusion, of a contrast agent (also referred to as contrast medium) within a patient or examination object.

In various examples, the contrast agent may be introduced into a patient and/or examination object, and subsequently, during an observation period, may be distributed or spread across at least part, e.g., an examination region, of the patient or examination object, by diffusion and/or perfusion. Accordingly, varying concentrations of the contrast agent are present in different parts of the examination region. Distribution of the contrast agent may be divided in different distribution phases, e.g., diffusion phases and/or perfusion phases. One of the distribution phases may refer to a phase before the contrast agent is introduced or before the contrast agent is distributed in the patient or examination object (pre-contrast). A point of time during the observation period may refer to a specific distribution of the contrast agent, or pre-distribution of the contrast agent, in other words to a specific distribution phase. The contrast agent concentration thus changes as a function of time and position.

In various examples, the processing of the ML model and/or the further ML model may be based on images acquired by a CE medical imaging system. Thus, in other words, the ML model and/or the further ML model, may obtain, as input data, CE images acquired using a CE measurement using the CE medical imaging system. Alternatively or additionally, the processing of the ML model and/or the further ML model may be based on raw or processed measurement data detected by the CE medical imaging system, for a contrast-enhanced measurement with multiple contrast agent distribution phases during an observation period. Alternatively or additionally, the methods may be based on measurement metadata associated with acquisition by the CE medical imaging system.

For instance, a pre-training image used for training the ML model may comprise at least one image acquired by the CE medical imaging system, in various examples at least two images, for example one pre-contrast image and one phase image, but also further available data as described.

In a step, a training of the ML model is performed. This training is done to reach a trained state for the ML model, prior to building the further ML model based on the ML model. Accordingly, this is referred to as "pre-training" (since it precedes the building of the further ML model). Here, the ML model is trained for predicting time-related information from at least one pre-training image acquired by the CE medical imaging system using the contrast-enhanced measurement, for the at least one pre-training image, in order to generate a pre-trained ML model.

In various examples, the pre-training may not require and/or use expert annotations in images. Unsupervised pre-training may be employed. This is because typically it is possible to provide ground truth for the time-related information in an automated fashion, e.g., without requiring expert annotation. This is explained in further detail hereinafter with respect to the nature of the time-related information.

The time-related information may be associated with one or more points of time during the observation period. Specifically, it is possible that the time-related information is associated with the distribution dynamics of the contrast agent throughout the patient during the observation period. It can be based on the pharmacokinetics of the contrast agent.

The time-related information may pertain to information that takes into account, i.e., is based on, information at multiple different points of time during the observation period. The time-related information may be time-dependent. The time-related information may be resolved in time domain in some examples; but, in other examples, does not need to be resolved in time domain. For instance, the time-related information could include a distribution of contrasts across different contrast agent distribution phases during the observation period. For instance, the time-related information can include an image acquired at a certain point in time during the observation period.

For example, the time-related information may be derivable from knowledge on the development of acquisitions (image and/or measurement data) over time, specifically over different contrast agent distribution phases. This can include differences in the images or measurement data of acquisitions at different points of time. The different point of time may be associated with different distribution phases or images.

In a step, said at least part of the pre-trained ML model is used for building (in other words generating) the further ML model for predicting semantic context information for at least one inference CE image.

The at least one inference CE image is acquired by the CE medical imaging system, e.g., using the CE measurement, or another CE measurement performed by the CE medical imaging system.

According to various examples, predicting semantic context information may be performed based on a single inference image, but also on multiple inference images: for example, if multiple inference images are available for multiple distribution phases, the further ML algorithm could be set up and in a way that it takes multiple inference images as input.

As a general rule, various options are available for building the further ML model based on the at least part of the pre-trained ML model. Some examples are disclosed below.

In an example, building the further ML model may refer to re-using and/or incorporating or including at least part of the pre-trained ML model into the further ML model. This means that, e.g., one or more layers of a neural-network implementation of the pre-trained ML model may be re-used in the further ML model. For instance, certain parameters of the pre-trained ML model that have been set during training of the pre-trained ML model may be reused for the further ML model. In one example, the at least the part of the pre-trained ML model may generate embedded features from the at least one inference image in the further ML model, based on which the semantic context information for the at least one inference image is determined. Thus, the at least part of the pre-trained ML model may be referred to as an ML "feature generator" that has been pre-trained and is used in the further ML model for processing images. This has the advantage that the pre-trained ML model is pre-trained to extract embedded features that are indicative or characteristic for the different distribution phases, i.e., a phase-dependent appearance of the CE image due to the pharmacokinetics of the contrast agent.

In various examples, building the further ML model for predicting the semantic context information may, for example, include supervised training of the at least part of the pre-trained ML model and re-using this at least part of the pre-trained ML model as the further ML model or as a part of the further ML model (as explained above). In detail, after the pre-training, the ML model can be in a first training state. This first training state may be reached based on unsupervised pre-training, to predict the time-related information. Starting from this first training state, it is then possible to perform supervised further training of the at least part of the pre-trained ML model. The pre-trained ML model is thus refined and brought to a second training state. In the second training state, the pre-trained ML model can be used as part of the further ML model. For instance, one or more layers of the pre-trained ML model in the second training state can be incorporated in the further ML model.

In a further example, the pre-trained ML model can be used to generate additional training data or information helpful for training the further ML model. The time-related information can be used when training the further ML model. For instance, images used for the training of the further ML model can be generated for further distribution phases using the ML model.

As will be appreciated, above, various options for building the further ML model based on a least part of the pre-trained ML model have been disclosed.

Next, details with respect to the pre-training of the ML model are disclosed. Specifically, techniques are disclosed that facilitate unsupervised pre-training. Various techniques are based on the finding that by not predicting the semantic context information (that is ultimately desired and predicted by the further ML model) by the ML model, but rather the time-related information, a - typically less complex - prediction task is enabled that facilitates the unsupervised training. Specifically, ground truth for the time-related information may be readily available.

The pre-training of the ML algorithm is generally based on at least one pre-training image. The at least one pre-training image have a different resolution and/or may be acquired using a different protocol as the inference images used to predict the semantic context information using the further ML algorithm.

The at least one pre-training image may be acquired by the CE medical imaging system at one or more first points of time during the observation period (e.g., before or during distribution of the contrast agent), and said time-related information may then information associated with an acquisition by the CE medical imaging system at one or more second points of time during the observation period. Hereinafter, for sake of simplicity reference is made to a (single) first point of time and a (single) second point of time, but respective techniques may be equally applicable to multiple points of time during the observation period.

By predicting the information associated with the second point in time based on the pre-training image(s) associated with the first point in time, unsupervised learning can be employed for the pre-training. For instance, the information associated with the second point in time could be determined by acquiring respective ground truth using the CE measurement that is also used to acquire respective at least one pre-training image at the first point in time. Alternatively or additionally, the information associated with the second point in time could be determined using a analytical model that predicts the distribution of contrast agent throughout an organ or the patient. Examples include Linear Time Invariant (LTI) models or compartment models that model pharmacokinetics of a contrast agent throughout the patient.

Said first point of time may be different from the second point of time, e.g., by a time offset by a defined time increment. For example, the first point in time can correspond to a pre-contrast phase, i.e., prior to a contrast agent being introduced into the patient. The second point in time, conversely, can correspond to a post-injection phase after the contrast agent is introduced into the patient.

In detail, it would be possible that the unsupervised pre-training of the ML model includes obtaining at least one pre-training image that is associated with the first point in time during the observation period. Then, the ML model can be applied to the at least one pre-training image so that the time-related information is predicted for the second point in time. Then, it would be possible to obtain ground truth information based on an acquired image at the second point in time and train the ML model based on comparing the predicted time-related information in the ground truth information.

In other words, additional measurement data may be acquired at the second point in time and the respective time-related information can be automatically derived therefrom, enabling unsupervised training. Manual annotations are not required. The ground truth information can be obtained through measurements.

For instance, a pre-contrast image may be used to predict a contrast-phase image (or vice versa). The pre-contrast image may be fed to the ML algorithm and the ML algorithm can predict the contrast-phase image. The respective ground truth contrast-phase image may also be acquired using the CE measurement and may then be compared to the prediction, to enable the unsupervised training.

Such implementation of the time-related information as an image acquired by the CE medical imaging system at the respective CE measurement is only one example. Other implementations of the time-related information associated with the one or more points of time during the observation period are possible. Further examples are given below:
In a first example, said time-related information may comprise, e.g., statistical information, such as a variance and/or standard deviation of image pixel intensities of at least one group of corresponding pixels across the observation period. Again, such information may be automatically derived from acquiring respective further pre-training images at one or more further points in time during the observation duration and then determining the statistical information therefrom. Unsupervised training is enabled.

In a second example, said time-related prediction information may comprise binary information for each of a group of pixels of the pre-training image. The time-related information can include a binary mask or a multi-level map that has, e.g., n of discrete values, e.g. n<10, or n<50, or n<100. Thereby, instead of a regression task, a discrete prediction can be implemented for the pre-trained ML algorithm, which further facilitates unsupervised training.

Said pre-trained ML model may comprise an autoencoder neural network architecture and/or a u-net neural network architecture.

An encoder branch of the pre-trained ML model may be used in the further ML model. Thereby, latent features can be extracted, the pre-trained ML model thus operating as a feature generator.

Said semantic context information may comprise information about presence of a region of interest, specifically a diseased region, in the image, and/or segmentation information related with a region of interest.

A computer-implemented method for predicting semantic context information from an image acquired by a CE medical imaging system using the further ML model is provided, wherein the further ML model has been trained according to any method according to the disclosed techniques.

The disclosed techniques enable an improved training of a ML model for determining semantic context information from an image generated by a CE imaging system, wherein less expert annotations and less computing and memory resources may be required compared to existing solutions. Further by the improved ML models, semantic context information may be extracted from an image with improved accuracy.

A computing device is provided comprising at least one processor and memory, the memory comprising instructions executable by the processor, wherein when executing the instructions in the processor, the computing device is configured to perform the steps of any method or combination of methods according to the present disclosure.

The computing device may, for example, comprise a computing device included in a medical imaging system, or an edge computing device on site of the medical imaging system and connected to the medical imaging hardware system by a communication network interface, or a remote computing device in a backend at a location remote from the medical imaging system or in a cloud computing service, e.g. a cloud computing device.

A computer program or a computer-program product and a computer-readable storage medium including program code is provided. The program code can be executed by at least one processor. Upon executing the program code, the at least one processor performs any method or combination of methods according to the present disclosure.

A medical system comprises at least one computing device according to the present disclosure.

For the electronic devices, computer program products, non-transitory computer-readable storage medium, and medical system, advantages may be realized, which correspond to the advantages described for the methods.

It is to be understood that the features mentioned above and features yet to be explained below can be used not only in the respective combinations indicated, but also in other combinations or in isolation, without departing from the scope of the present disclosure. In particular, features of the disclosed embodiments may be combined with each other in further embodiments.

The above summary is therefore only intended to give a brief overview of some features of some embodiments and implementations and is not to be understood as a limitation. Other embodiments may include features other than those described above.

The present disclosure is described in more detail below with reference to the accompanying drawings, in which like reference numerals refer to like elements.
FIG. 1 schematically illustrates a series of CE-MRI images acquired at different diffusion phases, according to various examples.
FIG. 2 schematically illustrates a series of CE-CT images acquired at different diffusion phases, according to various examples.
FIG. 3 schematically illustrates steps of a method for training a ML model based on images acquired by a CE medical imaging system, according to various examples.
FIG. 4 schematically illustrates a computing device configured for executing a method according to the present disclosure, according to various examples.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

Various techniques described herein generally relate to building a machine-learning (ML) model to determine semantic context information for images acquired using a CE measurement using a CE medical imaging system. Such building of the ML model can include setting parameters, obtaining appropriate parameters, executing a training, etc.

According to examples, the building of the ML model is facilitated by another pre-trained ML model. The pre-trained ML model can be pre-trained in an unsupervised manner, thereby enabling efficient training without a need for extensive expert annotation.

CE imaging uses the diffusion characteristics of contrast agents for improved visualization of normal or abnormal regions of interest (ROI) compared to the surrounding anatomy. This process normally involves the acquisition of a series of images of the ROI before and following the administration of a contrast agent in a chronological order. In other words, multiple CE images are acquired for multiple distribution phases during an observation duration.

Based on such CE images, various semantic context information can be determined. Some examples include lesion/tumor characterization for cancer staging, therapy or treatment planning using CE-CT, diagnosis of blood vessel conditions (aneurysms, blockages) using CT angiography, detection and characterization of brain abnormalities using CE-MRI.

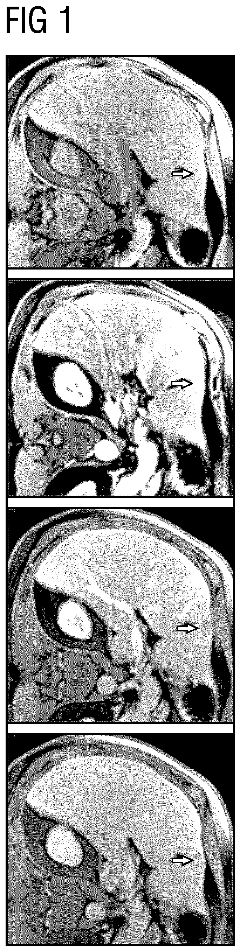

FIG. 1 schematically illustrates a series of four CE-MRI images acquired at different diffusion phases, according to various examples.

As can be seen in FIG. 1, an arrow points to a ROI, in which a diseased region is to be detected and segmented (as an example of semantic context information). The top image in FIG. 1, shows an image of a pre-contrast distribution phase, in which the diseased region is visible as a light shadow. The following image below, i.e., the second image from the top, shows an image in an arterial distribution phase of a contrast agent introduced into the patient. The following image below, i.e. the third image from the top, shows an image in a portal venous distribution phase, in which the diseased region is visible as a dark region compared to the background colour of the healthy tissue. The bottom image shows a late distribution phase of the contrast agent, in which the diseased region of interest is still visible as dark region.

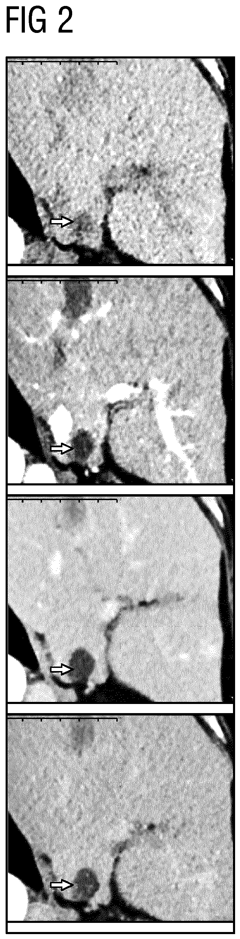

FIG. 2 schematically illustrates a series of four CE-CT images acquired at different diffusion phases, wherein the arrow points to a region of interest, according to various examples.

Similarly to FIG. 1, the top image in FIG. 2 shows an image of a pre-contrast phase, in which a diseased region is shown as a light grey shadow. The following image below, i.e. the second image from the top, shows an image in an arterial distribution phase of a contrast agent introduced into the patient, in which the diseased region of interest is shown as a darker region compared to the pre-contrast image. The following image below, i.e. the third image from the top, shows an image in a portal venous distribution phase, in which the diseased region is clearly visible as a dark region with sharp edges over the background colour of the healthy tissue. The bottom image shows delayed distribution phase of the contrast agent, in which the diseased region of interest is still visible as dark region.

As a general rule, as part of a CE measurement, CE images associated with different points of time during an observation period that are associated with different CE phases are chronologically acquired, typically starting with the pre-contrast phase. After the contrast agent is introduced, its diffusion/perfusion is tracked by acquiring measurement data and generating CE images at specific points of time during an observation period, for the different distribution phases, e.g., the arterial, portal venous and late/delayed phases, at time intervals as determined by the clinical protocol.

The disclosed techniques provide a learning method to build an ML model to predict semantic context information based on CE images (these CE images may be referred to as "inference images", because they are used to infer the semantic context information). To do this, another ML model is pre-trained to predict time-related information. Then, the actual ML model that predicts the semantic context information is built based on the pre-trained other ML model that implements the time-related prediction task.

Given two or more distribution phases this time-related prediction task may be implemented in multiple ways. For example, given one or more time-steps until a certain time point t during the observation period, it would be possible to predict the next single or more time step image values, t+1, t+2 ... (as an example of time-related information). To be specific, the pre-contrast image can be used as input to the ML model, and the ML model - in the current training state - can then predict one or more images at other distribution phases. These images can also be acquired using the CE measurement so that ground truth is directly available. Instead of predicting an image intensity value one could also apply a mask generation algorithm (e.g. thresholding) to turn the problem into a discrete prediction task. A more general setup using N pre-training images at multiple distribution phases would be to have N input pre-training images, which comprise distribution phase images of the different distribution phases. The input data for the ML model may the pre-training images, wherein one or more of the pre-training images at certain distribution phases are zeroed-out. The "pre-"task to be solved by the ML model is then to reconstruct the zeroed out input. By randomizing which distribution phase is zeroed out, a flexible ML model can be trained. Another possible embodiment is to not directly predict a single or more images at multiple distribution phases, but to rather predict the variance or standard deviation of intensities over the contrast phases, given a pre-contrast phase as input. Other statistical information may be predicted. It will be appreciated by a skilled person that, in order to train the ML model to predict time-related information, many more variations are possible. For example, prediction can also be setup to be backwards in time. The main purpose is to present a problem that requires a certain functional understanding of the imaged object as part of a solution algorithm. In liver imaging for example, contrast agent affects blood vessels, tumors and normal liver tissue differently over time.

An ML model that understands these tissue differences should therefore have generated features that can be useful for a different task such as the detection or segmentation or tumors in the liver or the detection of lesions in multi-phase coronary CTA.

The ML model that is pre-trained to predict the time-related information and used to build the further ML model that infers the semantic context information can be implemented, e.g., using an autoencoder or u-net type image to image neural network.

If there is significant motion between the phases a registration module can be employed as a preprocessing step, to establish pixel correspondences between the phases. Training can be performed with a sum of squared differences or sum of absolute differences loss in the case of intensity prediction and for example cross-entropy if one or more masks are to be predicted.

After pre-training, the autoencoder neural network (as an example implementation of the ML model) can be split into and encode and decoder branches. The encoder branch can then be used as feature extraction module for the downstream actual task (such as tumor segmentation). In other words, the encoder branch can be incorporated in to the further ML model that predicts the semantic context information. This is a "pre-training" setup.

The main part of this invention is the leveraging of the related problem of temporal prediction for the downstream task. This enables higher data efficiency with respect of the amount of annotated data there needs to be provided. Temporal prediction in multi-phase imaging offers a hard related problem where there is a lot of data for training available without having to annotate.

ROI appearance characteristics vary based on the phase as shown by the example in Fig. 1 and using an appropriate phase for detection/segmentation may help improve performance. Phase prediction, as described by this invention can be used to generate the appropriate phase in cases where it is unavailable due to the preexisting clinical protocol. In general, the methods according to the present disclosure allow to predict images at discrete time points (phases) and may be used as an "interpolation" method to generate time-resolved "videos" at any framerate. For example, when images of n phases at time t1, t2, ...tn are available, it would then be possible to predict arbitrary number of intermediate images between t1 and t2, t2 and t3, and similarly between any other sequential images, which may be combined into an animation/video showing effects of diffusion and/or perfusion at increased temporal resolution. Phase prediction, as disclosed herein allows downstream detection/segmentation tasks (implemented by the further ML algorithm) to leverage pretext features (i.e., latent features extracted using the upstream ML algorithm) that encode knowledge of the underlying anatomy to improve performance. As such, phase prediction can be used as a pretext task for ROI detection/segmentation.

Inclusion of the outcome and/or features generated by the phase prediction task therefore help the downstream detection/segmentation models converge with fewer expert annotations as reference or help improve model performance given the same number of expert annotations.

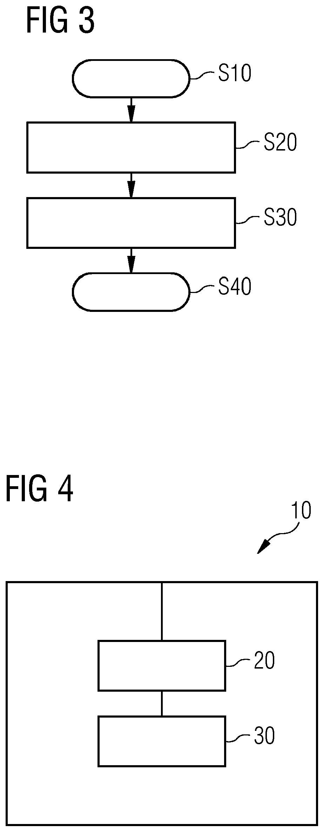

FIG. 3 schematically illustrates steps of a method for training a ML model based on images acquired by a CE medical imaging system for a contrast-enhanced measurement with multiple contrast agent distribution phases during an observation period, according to various examples.

For illustration, the method of FIG. 3 could be executed by the processor 20 of FIG. 4, upon loading program code from the memory 30, or by any other computing device, e.g. a computing device included in a medical imaging system, an edge device associated with a medical imaging system, or a backend computing device, such as a cloud computing device, remote to the medical imaging system.

The method starts in step S10.

In step S20, pre-training of a ML model is performed for predicting time-related information from at least one pre-training image acquired by the CE medical imaging system using the contrast-enhanced measurement, in order to generate a pre-trained ML model. The time-related information is associated with multiple points of time during the observation period.

The pre-training can be implemented in an unsupervised matter. This is because ground-truth information for the pre-training can be obtained from the CE measurement and/or from prediction of the pharmacokinetics of the contrast agent throughout the patient using another model, e.g., a compartment model.

In step S30, using at least part of the pre-trained ML model, a further ML model is built for predicting semantic context information from at least one inference image, the at least one inference image being acquired by the CE medical imaging system using the contrast-enhanced measurement. The method ends in step S40.

FIG. 4 schematically illustrates a computing device configured for executing a method according to the present disclosure, according to various examples.

The computing device 10 comprises at least one processor 20 and memory 30, the memory 30 comprising instructions executable by the processor 20, wherein when executing the instructions in the processor 20, the computing device 10 is configured to perform the steps according to any method or combinations of methods according to the present disclosure.

In particular, the processor 20 may obtain input data comprising e.g. at least one image generated by a medical imaging system via an interface, e.g., from a hospital database, a computer-readable storage medium, or directly from the imaging system. Upon loading program code from the memory 30, the processor 20 may process the input data to train an ML model, as described herein. Details with respect to such processing are illustrated in connection with FIG. 3.

Summarizing, various disclosed techniques rely on pre-training an ML model using unlabeled, unannotated image or measurement data; the pre-trained ML model is then used, in downstream processing, for a main task of predicting semantic context information in an image of a specific CE phase. A further ML model is built based on the pre-trained ML model.

According to examples, input data is processed using an ML algorithm and/or using trained functions. As a general rule, applying the ML model in the various examples may include applying a trained neural network, e.g., a deep-learning network. In various examples, processing the input dataset may comprise applying a trained ML model, i.e., trained network or a trained function, to the input dataset, wherein the ML model has been trained with training input datasets comprising images associated with different CE diffusion phases in image space, and corresponding known reference output data comprising further training images of specific CE diffusion phases. A trained function may for example comprise a neural network, wherein the input dataset is fed into the neural network in order to directly generate the output dataset. In various examples, applying the trained ML model may comprise applying an end-to-end trained ML network.

In various examples, a trained function may comprise an end-to-end trained function, which was trained with a plurality of training data sets. A training data set may include input data associated with reference output data. Applying trained functions may be performed, as some examples, by a neural network, which may comprise a plurality of classifier functions. In various examples, trained functions may comprise one or more of known machine learning classifiers. Without limitation, the trained functions may be based for example on one or more of a support vector machine, a decision tree and/or a Bayesian network, k-means clustering, Q-learning, genetic algorithms and/or association rules. For example, a neural network may comprise a deep neural network, a convolutional neural network, or a convolutional deep neural network, an adversarial network, a deep adversarial network and/or a generative adversarial network, or another machine-learning network architecture.

From the above said, the following general conclusions may be drawn:
Time-related information may refer to one or more missing images in sequential pre-training images associated with different distribution phases of a contrast agent. For example, a pre-training image may be associated with a point of time, time interval after introduction of the contrast agent, or by a diffusion phase or perfusion phase, during which the image was acquired. Based on at least one pre-training image, at least one further pre-training image may be predicted.

The at least one pre-training image and the at least one further pre-training image may be associated with different distribution phases.

For building the further ML model, the output data of the pre-training methods may be used. The further ML model is built based on the pre-trained ML model. The prediction tasks of the ML model and the further ML model differ in that the ML model predicts time-related information and the further ML model predicts semantic context information.

The further ML model may be used for tumor detection/segmentation in images, wherein the pre-training enables training the ML model for determining a temporal prediction with respect to time-based differences in images of different points of time in contrast-enhanced imaging methods. For example, a missing image of a specific diffusion phase could be predicted, and, in general, any statistical value, representing a time-based difference, for instance a difference between images taken at different points of time during or before the diffusion of a contrast-enhancing agent introduces into a patient, for corresponding pixels, that is to say any prediction that requires knowledge about the temporal evolution of diffusion phases. The ML model can then be further trained for tumor detection/segmentation, or parts of the model can be re-used in a further ML model for tumor detection/segmentation.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

For illustration, above, various scenarios have been disclosed in connection with CE Magnetic Resonance Imaging (MRI) techniques and CE Computed Tomography (CT) techniques. Similar techniques may be readily applied to various other kinds and types of digital imaging systems, for example medical imaging systems such as, for example, Microscopy, X-Ray, and Ultrasound Imaging (Sonography) systems, which may generate a plurality of images from measurement data of an examination object or patient, in which a contrast-enhancing agent, i.e. contrast agent, is introduced and dispersed.

## Claims

1. A computer-implemented method, the computer-implemented method comprising:
- unsupervised pre-training of a machine-learning, ML, model for predicting time-related information from at least one pre-training image acquired by a contrast-enhanced, CE, medical imaging system using a contrast-enhanced measurement of a patient with multiple contrast agent distribution phases during an observation period, in order to generate a pre-trained ML model, the time-related information being associated with one or more points of time during the observation period; and
- using at least part of the pre-trained ML model for building a further ML model for predicting semantic context information from at least one inference image acquired by the CE medical imaging system using the contrast-enhanced measurement or a further contrast-enhanced measurement.

2. The computer-implemented method of claim 1,
wherein said at least one pre-training image is acquired by the CE medical imaging system at a first point of time during the observation period, and
wherein said time-related information comprises information associated with an acquisition by the CE medical imaging system at a second point of time during the observation period, the second point of time being different than the first point of time.

3. The computer-implemented method of claim 1 or 2,
wherein said unsupervised pre-training of said ML model for predicting time-related information comprises:
- obtaining said at least one pre-training image of the patient acquired by the CE medical imaging system at a first point of time during the observation period;
- applying said ML model to the at least one pre-training image, whereby said time-related information is predicted for a second point of time during the observation period;
- obtaining ground-truth information based on a further image acquired by the CE medical imaging system at the second point of time; and
- training the ML model based on comparing the predicted time-related information and the ground-truth information.

4. The computer-implemented method of claim 2 or 3,
wherein the first point in time corresponds to a pre-contrast phase of the observation period prior to a contrast agent being introduced into the patient,
wherein the second point in time corresponds to a post-injection phase of the observation period after the contrast agent is introduced into the patient.

5. The computer-implemented method of any one of the preceding claims,
wherein the time-related information comprises at least one further pre-training image at the one or more points of time during the observation period.

6. The computer-implemented method of any one of the preceding claims,
wherein the time-related information comprises statistical information such as a variance and/or standard deviation of image pixel intensities across the observation period.

7. The computer-implemented method of any one of the preceding claims,
wherein the time-related information comprises a mask or a map for pixels of the at least one pre-training image.

8. The computer-implemented method of any one of the preceding claims,
wherein the pre-trained ML model comprises an autoencoder neural network architecture and/or a u-net neural network architecture.

9. The computer-implemented method of any one of the preceding claims,
wherein said using of said at least part of the pre-trained ML model for building the further ML model comprises:
- incorporating said at least part of the pre-trained ML model into the further ML model.

10. The computer-implemented method of claim 9, wherein said at least the part of the pre-trained ML model generates embedded features from the at least one inference image in the further ML model, based on which the semantic context information for the at least one inference image is determined.

11. The computer-implemented method of any one of the preceding claims,
wherein said using of said at least part of the pre-trained ML model for building said further ML model comprises:
- supervised training of said at least part of the pre-trained ML model using further training images which are annotated with ground-truth semantic context information.

12. The computer-implemented method of any one of the preceding claims,
wherein the semantic context information comprises information about presence of a region of interest, specifically a diseased region, in the inference image, and/or segmentation information related with a region of interest.

13. A computer-implemented method for predicting semantic context information from an image acquired by a CE medical imaging system using the further ML model which has been built according to the method of one of the preceding claims.

14. Computing device (10) comprising a processor (20) and memory (30), the memory (30) comprising instructions executable by the processor (20), wherein when executing the instructions in the processor (20), the computing device (10) is configured to perform the computer-implemented method of one of claims 1 to 13.

15. Medical imaging system comprising at least one computing device according to claim 14.

16. A computer program or a computer-readable storage medium comprising instructions which, when the program is executed by a computer, cause the computer to carry out the method of one of claim 1 to 13.
